# EUROPÄISCHE PATENTSCHRIFT

(11) **EP 0 648 735 B1**
(45) Veröffentlichungstag und Bekanntmachung des Hinweises auf die Patenterteilung: **28.01.1998**
(21) Anmeldenummer: 94115331.4
(22) Anmeldetag: 29.09.1994
(51) Int. Cl.: C07C 69/007, C07C 45/40, C07C 45/62, C07C 47/263, C07C 403/12, C07C 409/20

(54) **Herstellung eines Zwischenproduktes zum Vitamin A-Acetat**
Preparation of an intermediate for vitamin A-acetate
Préparation d'un intermédiaire pour l'acétate de la vitamine A

(30) Priorität: 13.10.1993 CH 3077/93
(43) Veröffentlichungstag der Anmeldung: 19.04.1995
(73) Patentinhaber: F. HOFFMANN-LA ROCHE AG, 4002 Basel (CH)
(72) Erfinder: Zutter, Ulrich, CH-4051 Basel (CH)
(74) Vertreter: Kellenberger, Marcus, Dr.

(56) Entgegenhaltungen:
- BE-A- 564 339
- FR-A- 1 576 842
- FR-A- 2 266 689
- FR-A- 2 383 908

## Beschreibung

Die vorliegende Erfindung betrifft ein neues Verfahren zur Herstellung von γ-Acetoxytiglinaldehyd (dem "C₅-Aldehyd"; I), der als Ausgangsmaterial für die Herstellung von Vitamin A-Acetat aus einem (β-Jonylidenäthyl)triphenylphosphoniumhalogenid (dem "C₁₅-Vinylsalz") und diesem C₅-Aldehyd bekannt ist.

In der Schlussstufe der bekannten Vitamin A-Acetat-Synthese wird mittels einer Wittig-Reaktion der C₅-Aldehyd mit dem C₁₅-Vinylsalz gekoppelt. Dabei entsteht direkt das wegen seiner besonders ausgeprägten biologischen Aktivität bevorzugte all-trans-Vitamin A-Acetat mit hoher Selektivität. Als aufwendig und zu kostspielig erweist sich hingegen bei dieser Wittig-Synthese noch immer die Herstellung des C₅-Aldehyds.

Das erfindungsgemässe Verfahren zur Herstellung von γ-Acetoxytiglinaldehyd ist dadurch gekennzeichnet, dass man ein Pentenin-3-ol der allgemeinen Formel

HC≡C-C(CH₃)(OH)-CH=C(R)₂ II

worin beide R entweder Wasserstoff oder Methyl bedeuten, einer selektiven Ozonolyse in einem niederen Alkanol R¹OH, worin R¹ C₁₋₄-Alkyl bedeutet, unterwirft, das so erhaltene 2-Hydroxy-2-methyl-3-butinal der Formel

HC≡C-C(CH₃)(OH)-CHO III

sowie allfällig als Nebenprodukt erhaltenes 1-Alkoxyalkyl-hydroperoxid der allgemeinen Formel 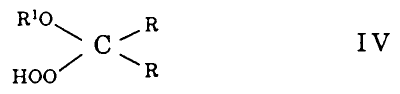 worin beide R entweder Wasserstoff oder Methyl und R¹ C₁₋₄-Alkyl bedeuten,
zum bekannten 2-Hydroxy-2-methyl-3-butenal der Formel

H₂C=CH-C(CH₃)(OH)-CHO V

katalytisch (und im Falle des 2-Hydroxy-2-methyl-3-butinals der Formel III auch selektiv) hydriert und dieses auf an sich bekannte Weise in den gewünschten γ-Acetoxytiglinaldehyd der Formel

CH₃COO-CH₂CH=C(CH₃)-CHO I

überführt.

Die Ozonolyse der ersten Verfahrensstufe erfolgt zweckmässigerweise in einem niederen Alkanol R¹OH, vorzugsweise Methanol oder Aethanol, insbesondere in Methanol, als Lösungsmittel und bei relativ niedrigen Temperaturen bis etwa 0°C, vorzugsweise bei Temperaturen im Bereich von etwa -20°C bis etwa 0°C. Das Ozon wird zweckmässigerweise in die auf der niedrigen Temperatur gehaltene Reaktionslösung solange eingeleitet, bis ein (nahezu) vollständiger Umsatz, z.B. nach Gaschromatographie, festgestellt worden ist. Zudem empfiehlt es sich, nach Beendigung der Ozonolyse die Ozonolyselösung mit einem Inertgas, z.B. Argon, zu begasen, um überschüssiges Ozon zu entfernen. Bei der Reaktion wird neben dem 2-Hydroxy-2-methyl-3-butinal der Formel III auch das 1-Alkoxyalkyl-hydroperoxid der Formel IV erzeugt. Zudem kann es vorkommen, dass infolge der Verwendung des niederen Alkanols R¹OH als Lösungsmittel bei der Ozonolyse das 2-Hydroxy-2-methyl-3-butinal teilweise in das entsprechende Halbacetal der Formel

HC≡C-C(CH₃)(OH)-CH(OH)(OR¹)

umgewandelt wird. Sowohl das 1-Alkoxyalkyl-hydroperoxid als auch das Halbacetal können zusammen mit dem Hauptprodukt, d.h. dem 2-Hydroxy-2-methyl-3-butinal, unmittelbar in der anschliessenden katalytischen Hydrierung umgesetzt werden, ohne dass das eine oder beide Nebenprodukte zuerst entfernt werden muss bzw. müssen.

Es hat sich gezeigt, dass die anschliessende Hydrierung des 2-Hydroxy-2-methyl-3-butinals und von allfällig vorhandenem Halbacetal, wobei die (jeweilige) Dreifachbindung selektiv zur Doppelbindung reduziert wird, sowie die gleichzeitige hydrogenolytische Spaltung des 1-Alkoxyalkyl-hydroperoxids zweckmässigerweise durch Zugabe der noch kalten Ozonolyselösung zum Katalysator in Suspension unter Einleitung des Wasserstoffs erfolgt. Als Katalysator eignet sich insbesondere ein Platin-oder Palladiumkatalysator. Der Palladiumkatalysator, z.B. Palladium auf Aluminiumoxid oder Palladium auf Kohle, besitzt eine grössere Selektivität gegenüber Platinkatalysatoren und ist deshalb bevorzugt. Der Katalysator wird zweckmässigerweise vorhydriert, und zwar im entsprechenden Alkanol R¹OH, z.B. Methanol, bei Raumtemperatur. Dadurch entsteht die Katalysatorsuspension, zu der die kalte Ozonolyselösung anschliessend zugegeben wird. Diese Zugabe wird vorzugsweise tropfenweise durchgeführt: auf diese Weise wird die sonst oft beobachtete, Hydroperoxiden zugeschriebene Vergiftung des Katalysators vermieden. Zudem wird die Dosierung während der Hydrierung so gesteuert, dass die Peroxidkonzentration möglichst klein bleibt. Ebenfalls zu beachten ist, dass während der Hydrierung das Butinal nie vollständig abreagiert, da sonst auch überhydriertes Produkt, nämlich 2-Hydroxy-2-methyl-butanal, entsteht. Dies wird erreicht, indem man die einzelnen Portionen der Ozonolyselösung jeweils nur bis zu einem Restgehalt am Butinal hydriert. Die Hydrierung erfolgt zweckmässigerweise bei Normaldruck oder einem Ueberdruck von bis zu etwa 20 bar sowie bei Temperaturen zwischen etwa 20°C und etwa 40°C, vorzugsweise bei Raumtemperatur. Nach Beendigung der Wasserstoffaufnahme wird der Katalysator entfernt, z.B. durch Filtration. Die Isolierung des so hergestellten 2-Hydroxy-2-methyl-3-butenals erweist sich als unnötig, da dessen Alkohollösung unmittelbar in der nächsten Verfahrensstufe behandelt werden kann. Das Gleiche gilt für allfällig vorhandenes Halbacetal dieser Verbindung, das durch katalytische, selektive Hydrierung des bei der Ozonolyse entstandenen Butinal-Halbacetals erhalten wurde.

In der belgischen Patentschrift 564.339 sind auch Reaktionsbedingungen für die an sich bekannte selektive Hydrierung des 2-Hydroxy-2-methyl-3-butinals der Formel III zum entsprechenden 3-Butenal der Formel V beschrieben. In diesem Fall wird allerdings das Butinal der Formel III seinerseits durch Aethinylierung (Umsetzung mit Acetylen) von Methylglyoxalacetal erhalten.

Wie oben erwähnt, kann das in der zweiten Verfahrensstufe erhaltene 2-Hydroxy-2-methyl-3-butenal der Formel V auf an sich bekannte Weise in den gewünschten γ-Acetoxyliglinaldehyd der Formel I übergeführt werden. Dies erfolgt zweckmässigerweise, indem man das Butenal [sowie allfällig vorhandenes Halbacetal davon, der Formel H₂C=CH-C(CH₃)(OH)-CH(OH)(OR¹)] zum entsprechenden Dialkylacetal der Formel

H₂C=CH-C(CH₃)(OH)-CH(OR²)₂ VI

worin R² C₁₋₄-Alkyl bedeutet,
acetalisiert [bzw. zum Dialkylacetal der Formel H₂C=CH-C(CH₃)(OH)-CH(OR¹)(OR²) VI' vollacetalisiert], das Dialkylacetal acetyliert, das resultierende 2-Acetoxy-2-methyl-3-butenal-dialkylacetal der Formel

H₂C=CH-C(CH₃)(OCOCH₃)-CH(OR²)₂ VII

[bzw. H₂C=CH-C(CH₃)(OCOCH₃)-CH(OR¹)(OR²)] VII'

einer katalysierten Umlagerung zum γ-Acetoxytiglinaldehyd-dialkylacetal der Formel

CH₃COO-CH₂CH=C(CH₃)-CH(OR²)₂ VIII

[bzw. CH₃COO-CH₂CH=C(CH₃)-CH(OR¹)(OR²)] VIII'

unterwirft und dieses mittels säurekatalysierter Hydrolyse in den gewünschten γ-Acetoxytiglinaldehyd der Formel I überführt.

Zur Durchführung der Acetalisierung verwendet man als Acetalisierungsmittel vorzugsweise Acetondimethylacetal, und zwar zweckmässigerweise bei einem etwa 1-molaren Ueberschuss bezogen auf die eingesetzte Menge Pentenin-3-ol der Formel II. Die Umsetzung erfolgt zudem in Gegenwart einer Säure, vorzugsweise Salzsäure oder Schwefelsäure, und - wie oben erwähnt - unter Einsatz der alkoholischen Produktlösung der Vorstufe. Die Acetalisierung erfolgt zweckmässigerweise bei erhöhter Temperatur, vorzugsweise bei der Rückflusstemperatur des Reaktionsgemisches. Das Produkt, das 2-Hydroxy-2-methyl-3-butenal-dimethylacetal, kann auf an sich bekannte Weise isoliert und gereinigt werden, beispielsweise durch Neutralisieren des Gemisches mit einer Base, z.B. Natriummethylat, Abdestillieren des Lösungsmittels und schliesslich Destillieren des Rückstandes unter vermindertem Druck.

Die Acetalisierung kann auch durch an sich bekannte säurekatalysierte Umsetzung des Butenals und eventuell Halbacetals mit dem entsprechenden niederen Alkanol R²OH durchgeführt werden, wobei als saurer Katalysator Salzsäure bevorzugt ist.

Die anschliessende Acetylierung kann ebenfalls in an sich bekannter Weise erfolgen, beispielsweise unter Verwendung eines entsprechenden Acetylhalogenid, insbesondere des Chlorids, oder von Essigsäureanhydrid. Man führt die Acetylierung vorzugsweise mit einem Ueberschuss an siedendem Essigsäureanhydrid durch, wobei die entstehende Essigsäure laufend und rasch aus dem Reaktionsgemisch herausdesfilliert wird. Auf diese Weise verläuft die Acetylierung nahezu quantitativ. Je nach Apparatur verwendet man zweckmässigerweise einen Ueberschuss an Säureanhydrid, und zwar insbesondere etwa 2 bis etwa 10 Mol Säureanhydrid pro Mol Dialkylacetal VI (und VI'). Das acetylierte Produkt lässt sich nach der Reaktion aus dem Reaktionsgemisch auf konventionelle Weise isolieren, und kann auch auf übliche Weise gereinigt werden.

Die nächste Verfahrensstufe, die katalysierte Umlagerung des 2-Acetoxy-2-methyl-3-butenal-dialkylacetals der Formel VII (und eventuell auch VII'), kann beispielsweise gemäss der in der DOS 1.297.597 oder in der DOS 2.840.125 beschriebenen und exemplifizierten Methode durchgeführt werden, und zwar durch Erhitzen des Dialkylacetals VII bzw. VII' in Gegenwart von metallischem Kupfer oder einer Kupferverbindung, insbesondere Kupfer(I)chlorid, als Katalysator bei Temperaturen von 50 bis 250°C, vorzugsweise 110 bis 180°C. Das Kupfer(I)chlorid kann allein oder auf einem inerten Trägermaterial getragen eingesetzt werden und wird im allgemeinen in Mengen von 0,005 bis 5 Gewichtsprozent, zweckmässigerweise 0,05 bis 0,5 Gewichtsprozent, berechnet als Kupfer bezogen auf das Ausgangsmaterial, verwendet. Die während der Umlagerung entstehenden niedersiedenden Nebenprodukte können vom Reaktionsgemisch durch Abdestillieren, vorzugsweise kontinuierlich, bei Normaldruck oder vermindertem Druck, zweckmässigerweise bei einem Druck von 100 bis 500 mbar, vorzugsweise 200 bis 350 mbar, oder alternativ durch Strippen mit einem das Reaktionsgemisch durchströmenden Inertgas, z.B. Stickstoff oder Argon, oder sogar Kohlendioxid, Methan, Wasserstoff oder Methylchlorid, abgetrennt werden.

Als Alternative zum obigen Umlagerungsverfahren kann man die in der DOS 2.513.198 beschriebene und exemplifizierte Methode verwenden. Bei dieser Methode erfolgt die Umlagerung in Gegenwart eines Palladiumkatalysators der allgemeinen Formel

(PdX₂Y)ₙ

worin n 1 oder 2 und X Chlor oder Brom bedeuten und, falls n 1 ist, Y die Gruppe (R³CN)₂ bedeutet, worin R³ eine Alkyl- oder Arylgruppe darstellt, oder falls n 2 ist, Y einen einfach olefinisch ungesättigten aliphatischen oder cycloaliphatischen Kohlenwasserstoffrest bedeutet,
vorzugsweise von Bis-(acetonitril)-palladiumchlorid oder -palladiumbromid der Formel PdX₂(CH₃CN)₂, insbesondere dem ersteren, d.h. der Formel PdCl₂(CH₃CN)₂. Die Umlagerung kann ohne oder mit Verwendung eines Lösungsmittels durchgeführt werden, wobei als Lösungsmittel insbesondere chlorierte Kohlenwasserstoffe, wie chlorierte Aethylene, z.B. Di-, Tri- und Tetrachloräthylen; niedere aliphatische Ketone, z.B. Aceton; niedere aliphatische oder zyklische Aether, z.B. Diisopropyläther bzw. Dioxan; sowie niedere aliphatische Ester, z.B. Aethylacetat, in Betracht kommen. Zudem erfolgt die Umlagerung zweckmässigerweise in einem Temperaturbereich zwischen etwa Raumtemperatur und etwa 100°C, insbesondere zwischen etwa 30 und etwa 50°C. Die Katalysatormenge kann von weniger als 1 Gewichtspromille zu bis etwa 7-8 Gewichtspromille, bezogen auf die Menge verwendeten Ausgangsmaterials, schwanken. Je nach verwendeter Reaktionstemperatur und der verwendeten Katalysatormenge liegt die Reaktionszeit zwischen wenigen Minuten und mehreren Stunden, z.B. etwa 20 Stunden. Die Umlagerung und die darauf folgende säurekatalysierte Hydrolyse zum γ-Acetoxytiglinaldehyd ist in Beispiel 1 der DOS 2.513.198 exemplifiziert.

Im allgemeinen ist die Methodik der DOS 2.513.198 [Verwendung eines Palladiumkatalysators (PdX₂Y)ₙ] gegenüber derjenigen der DOS 1.297.597 oder DOS 2.840.125 [Verwendung von metallischem Kupfer oder einer Kupferverbindung, insbesondere Kupfer(I)chlorid] bevorzugt. In jedem Fall kann die Isolierung des Umlagerungsproduktes und dessen Reinigung nach an sich bekannten Methoden erfolgen.

Die Endstufe zum gewünschten Produkt γ-Acetoxytiglinaldehyd CH₃COO-CH₂CH=C(CH₃)-CHO (I) kann durch saure Hydrolyse in an sich bekannter Weise erfolgen, beispielsweise durch Behandlung des γ-Acetoxytiglinaldehyd-dialkylacetals (VIII) mit einer verdünnten Mineralsäure, z.B. Salzsäure, zweckmässigerweise bei Raumtemperatur. Auch in diesem Fall kann das Produkt auf an sich bekannte Weise isoliert und gereinigt werden.

Die Erfindung wird durch die nachfolgenden Beispiele veranschaulicht.

### Beispiel 1

### Herstellung von 3,5-Dimethyl-4-hexen-1-in-3-ol (Ausgangsmaterial für die Ozonolyse)

In einem mit Rührer, Gaseinleitungsrohr, Thermometer und Kohlendioxid/Aceton-Kühler versehenen 4,5 L-Sulfierkolben werden unter Kühlung mit einem Kohlendioxid/Aceton-Bad bei -40°C 1,875 L Ammoniak bei einer Innentemperatur von etwa -34°C vorgelegt. Anschliessend werden 26,03 g (3,75 Mol) Lithiumdraht in Stücken von 2-3 cm Länge innert 45 Minuten unter Rühren zugegeben, und die resultierende dunkelblaue Lösung wird 15 Minuten nachgerührt. Bei einer Innentemperatur von -34°C (Badtemperatur -42 bis -44°C) wird nun Acetylen bei einer Durchlaufgeschwindigkeit von 4 L/Min. durch das Gaseinleitungsrohr in die Ammoniaklösung eingeleitet. Nachdem nach 45 Minuten ein Farbumschlag von dunkelblau nach grau (durchsichtig) erfolgt, wird noch in den folgenden 30 Minuten weiteres Acetylen eingeleitet, wonach der Acetylenverbrauch etwa 300 L beträgt. Dabei sinkt die Innentemperatur auf -37°C bei einer Badtemperatur von -35°C.

Das Aceton im Kühler wird nun von -78°C auf -20°C aufgewärmt und das Kohlendioxid/Aceton-Bad durch ein Wasserbad bei 30-40°C ersetzt. Während auf diese Weise das Ammoniak abdestilliert, wird das Volumen des Reaktionsgemisches durch kontinuierliches Zutropfen von insgesamt etwa 1,875 L Diäthyläther konstant gehalten. Dabei steigt die Innentemperatur von -37°C auf +5°C an, und aus dem grauen Reaktionsgemisch entsteht eine weisse Suspension. Man hält das Reaktionsgemisch 15 Minuten bei 5°C und sättigt es ohne Wasserbad 30 Minuten mit 2 L/Min. Acetylen nach. Unter ständigem Einleiten von Acetylen (2 L/Min.) wird nun auf -10°C abgekühlt und bei dieser Temperatur eine Lösung von 294,5 g (3 Mol) Mesityloxid in 255 ml Diäthyläther innert 50 Minuten bei einer Badtemperatur von etwa -30°C zugetropft. Anschliessend wird 10 Minuten nachgerührt und das Acetyleneinleiten abgestellt, da gemäss Gaschromatographie (GC) der Umsatz dann vollständig ist.

Zur Neutralisation werden nun innert 3 Stunden etwa 870 ml 30%ige Schwefelsäure bei -5 bis +5°C bis zu einem pH-Wert von 7 des Reaktionsgemisches langsam und unter intensivem Rühren zugetropft, während-dessen die Badtemperatur bei -30°C gehalten wird. Dann wird in einem Scheidetrichter die Wasserphase abgetrennt. Man trocknet die organische Phase über wasserfreiem Natriumsulfat (100 g) und nach Abfiltration des Trocknungsmittels das Lösungsmittel unter vermindertem Druck bei 30°C abdampft. Der Rückstand (364,3 g, 97,8% der theoretischen Ausbeute) wird einer fraktionierten Destillation unterworfen: die relevanten Angaben sind in der nachfolgenden Tabelle zusammengefasst.

| **Teil des Rohprodukts** | **Badtemperatur, °C** | **Sdp., °C** | **Druck, mbar** | **Gewicht** | **Reinheit gemäss GC** |
|---|---|---|---|---|---|
| Vorlauf | 65-70° | 46-65° | 23 | 6,5 g | 92,4 F% |
| Hauptfraktion | 74-80° | 66-69° | 20 | 332,2 g | 98,6 F% |
| Rückstand | | | | 16,1 g | 70,7 F% |

Die Hauptfraktion ergibt 332,2 g (89,2% der theoretischen Ausbeute) leicht gelbliche Flüssigkeit, Sdp. 66-69°/20 mbar, welche nach Gaschromatographie (GC) zu 98,6 F% aus dem gewünschten 3,5-Dimethyl-4-hexen-1-in-3-ol besteht.

### Beispiel 2

### Herstellung von 2-Hydroxy-2-methyl-3-butinal (Formel III)

In einem mit Rührer, Thermometer, Gaseinleitungsrohr und Blasenzähler versehenen 350 ml-Kühlmantelkolben wird unter Rühren durch eine Lösung von 49,7 g (400 mMol) 3,5-Dimethyl-4-hexen-1-in-3-ol (GC 98,6 F%; gemäss Beispiel 1 hergestellt) in 200 ml Methanol bei -20°C Ozon 5 Stunden lang eingeleitet. Während der Ozonleitung wird überschüssiges Ozon über den Blasenzähler in den Abzug geleitet. Innert dieser Periode erfolgt gemäss Gaschromatographie der vollständige Umsatz. Zur Entfernung des überschüssigen Ozons wird die Ozonolyselösung bei -20°C während 30 Minuten mit Argon begast. Diese Lösung wird anschliessend in einen auf -20°C gekühlten 250 ml-Kühltropftrichter transferiert und mit wenig Methanolspülungen auf 250 ml verdünnt. Das im Methanol gelöste Reaktionsprodukt besteht gemäss Gaschromatographie hauptsächlich aus dem gewünschten 2-Hydroxy-2-methyl-3-butinal. Als Nebenprodukt wird 1-Methoxy-1-methyläthyl-hydroperoxid nachgewiesen.

### Beispiel 3

### Herstellung von 2-Hydroxy-2-methyl-3-butenal (Formel V)

In einem mit graduiertem 250 ml-Kühltropftrichter (mit Druckausgleich), Begasungsrührer, Thermometer und Septum (zur Probenahme) versehenen 1,5 L-Sulfierkolben werden 10 g 5%iges Palladium auf Aluminiumoxid in 800 ml Methanol 20 Minuten bei Raumtemperatur vorhydriert. Nun werden 25 ml Ozonolyselösung (siehe Beispiel 2) zur Katalysatorsuspension getropft und anschliessend bis zur Aufnahme von 1,4 L Wasserstoff bei Normaldruck hydriert. Mit Hilfe der gaschromatographischen Analytik wird ein minimaler Restgehalt an 2-Hydroxy-2-methyl-3-butinal von 2-20% sichergestellt, um das Entstehen überhydrierten Produktes zu vermeiden. Die restliche Ozonolyselösung wird in neun 25 ml Portionen zugegeben und jeweils bei etwa 30°C (auf welche Temperatur sich das Reaktionsgemisch von selbst erwärmt) bis zur Aufnahme von 1,7 L Wasserstoff hydriert. Wie bisher wird der minimale Restgehalt an 2-Hydroxy-2-methyl-3-butinal von 2-20% sichergestellt. Die Hydrierdauer der 25 ml Portionen vergrössert sich von 10 Minuten für die 1. auf 50 Minuten für die 10. Portion, was eine Gesamthydrierzeit von 6 Stunden ergibt. Nach der Wasserstoffaufnahme von insgesamt 16,8 L (Theorie 19,3 L; bei der die GC-Analytik kein Butinal mehr und weniger als 0,5%ige Ueberhydrierung nachweist) wird der Katalysator über etwa 20 g Dicalite Speedex (Filtrationsmittel) abfiltriert und mit 100 ml Methanol gewaschen. Das Filtrat (etwa 1,2 L) enthält das gewünschte 2-Hydroxy-2-methyl-3-butenal, welches gemäss jodometrischer Titration einer 1 ml-Probe mit keinem Peroxid begleitet ist.

### Beispiel 4

### Herstellung von 2-Hydroxy-2-methyl-3-butenal-dimethylacetal (Formel VI)

In einem mit Rührer, Thermometer und Rückflusskühler versehenen 1,5 L-Sulfierkolben wird das Filtrat der letzten Verfahrensstufe (etwa 1,2 L; siehe Beispiel 3) mit 83,3 g (800 mMol, 98 ml) Aceton-dimethylacetal versetzt. Anschliessend wird der pH-Wert mit 1,0 ml 37%iger Salzsäure von etwa 4,5 auf etwa 1 eingestellt und das Reaktionsgemisch 2,5 Stunden auf Rückflusstemperatur bei einer Badtemperatur von 85°C erhitzt. Danach wird das Gemisch auf Raumtemperatur abgekühlt und der pH-Wert mit etwa 2,5 ml 30%igem Natriummethylat in Methanol auf 7-8 eingestellt. Man destilliert das Lösungsmittel (Methanol mit wenig Aceton) über eine mit Wilsonspiralen gefüllte 50 cm-Füllkörperkolonne (⌀ 2,5 cm) bei Normaldruck, einer Kopftemperatur von 62-65°C und einer Oelbadtemperatur von 90-95°C ab und unterwirft den Rückstand (81 g) einer fraktionierten Destillation unter vermindertem Druck (Wasserstrahlvakuum) über eine versilberte, mit Wilsonspiralen gefüllte 10 cm-Füllkörperkolonne (⌀ 1,5 cm). Folgende Fraktionen werden entnommen:

| **Fraktion** | **Badtemperatur, °C** | **Sdp., °C** | **Druck, mbar** | **Gewicht** | **GC F% Produkt** |
|---|---|---|---|---|---|
| 1 | 55° | 42-27° | 10³-60 | 24,6 g | 0,0 |
| 2 | 55-72° | 25-62° | 19 | 1,4 g | 73,6 |
| 3 | 69-135° | 63-65° | 19 | 41,4 g | 97,0 |
| Rückstand | | | | 9,4 g | |

Die Hauptfraktion (3) ergibt 41,4 g (70,8% der theoretischen Ausbeute) farblose Flüssigkeit, Sdp. 63-65°C/19 mbar, welche nach Gaschromatographie zu 97,0 F% aus dem erwünschten Produkt 2-Hydroxy-2-methyl-3-butenal-dimethylacetal besteht.

### Beispiel 5

### Herstellung von 2-Hydroxy-2-methyl-3-butinal (Formel III)

In einem mit Rührer, Thermometer, Gaseinleitungsrohr und Blasenzähler versehenen 350 ml-Kühlmantelkolben wird unter Rühren durch eine Lösung von 38,45 g (400 mMol) 3-Hydroxy-3-methyl-1-penten-4-in (GC 98,0 F%) in 200 ml Methanol bei -20°C Ozon 5,5 Stunden lang eingeleitet. Während der Ozonleitung wird überschüssiges Ozon über den Blasenzähler in den Abzug geleitet. Innert dieser Periode erfolgt gemäss Gaschromatographie praktisch der vollständige Umsatz. Zur Entfernung des überschüssigen Ozons wird die Ozonolyselösung bei -20°C während 30 Minuten mit Argon begast. Diese Lösung wird anschliessend in einen auf -20°C gekühlten 250 ml-Kühltropftrichter transferiert und mit wenig Methanolspülungen auf 225 ml verdünnt. Das im Methanol gelöste Reaktionsprodukt besteht gemäss Gaschromatographie hauptsächlich aus dem gewünschten 2-Hydroxy-2-methyl-3-butinal. Als Nebenprodukt wird Methoxymethylhydroperoxid nachgewiesen.

### Beispiel 6

### Herstellung von 2-Hydroxy-2-methyl-3-butenal (Formel V)

In einem mit graduiertem 250 ml-Kühltropftrichter (mit Druckausgleich), Begasungsrührer, Thermometer und Septum (zur Probenahme) versehenen 1,5 L-Sulfierkolben werden 7,7 g 5%iges Palladium auf Aluminiumoxid in 800 ml Methanol 20 Minuten bei Raumtemperatur vorhydriert. Nun werden 25 ml Ozonolyselösung (siehe Beispiel 5) zur Katalysatorsuspension getropft und anschliessend bis zur Aufnahme von 1,4 L Wasserstoff bei Normaldruck hydriert. Mit Hilfe der gaschromatographischen Analytik wird ein minimaler Restgehalt an 2-Hydroxy-2-methyl-3-butinal von 2-20% sichergestellt, um das Entstehen überhydrierten Produktes zu vermeiden. Die restliche Ozonolyselösung wird in neun 25 ml Portionen zugegeben und jeweils bei etwa 30°C (auf welche Temperatur sich das Reaktionsgemisch von selbst erwärmt) bis zur Aufnahme von 1,5 L Wasserstoff hydriert. Wie bisher wird der minimale Restgehalt an 2-Hydroxy-2-methyl-3-butinal von 2-20% sichergestellt. Die Hydrierdauer der 25 ml Portionen vergrössert sich von 15 bis 25 Minuten, was eine Gesamthydrierzeit von 3,5 Stunden ergibt. Nach der Wasserstoffaufnahme von insgesamt 13,5 L (Theorie 19,3 L; bei der die GC-Analytik kein Butinal mehr und nur etwa 1,5%ige Ueberhydrierung nachweist) wird der Katalysator über etwa 20 g Dicalite Speedex (Filtrationsmittel) abfiltriert und mit 100 ml Methanol gewaschen. Das Filtrat (etwa 1,2 L) enthält das gewünschte 2-Hydroxy-2-methyl-3-butenal, welches gemäss jodometrischer Titration einer 1 ml-Probe mit keinem Peroxid begleitet ist.

### Beispiel 7

### Herstellung von 2-Hydroxy-2-methyl-3-butenal-dimethylacetal (Formel VI)

In einem mit Rührer, Thermometer und Rückflusskühler versehenen 1,5 L-Sulfierkolben wird das Filtrat der letzten Verfahrensstufe (etwa 1,2 L; siehe Beispiel 6) mit 125 g (1200 mMol, 147,2 ml) Aceton-dimethylacetal versetzt. Anschliessend wird der pH-Wert mit 0,5 ml konzentrierter Schwefelsäure von etwa 4,5 auf 1-2 eingestellt und das Reaktionsgemisch 3 Stunden auf Rückflusstemperatur bei einer Badtemperatur von 85°C erhitzt. Danach wird das Gemisch auf Raumtemperatur abgekühlt und der pH-Wert mit 3 ml 30%igem Natriummethylat in Methanol auf 7-8 eingestellt. Man destilliert das Lösungsmittel (Methanol mit wenig Aceton) über eine mit Wilsonspiralen gefüllte 50 cm-Füllkörperkolonne (⌀ 2,5 cm) bei Normaldruck, einer Kopftemperatur von 62-65°C und einer Oelbadtemperatur von 90-95°C ab und unterwirft den Rückstand einer fraktionierten Destillation unter vermindertem Druck (Wasserstrahlvakuum) über eine versilberte, mit Wilsonspiralen gefüllte 10 cm-Füllkörperkolonne (⌀ 1,5 cm). Folgende Fraktionen werden entnommen:

| **Fraktion** | **Badtemperatur, °C** | **Sdp., °C** | **Druck, mbar** | **Gewicht** | **GC F% Produkt** |
|---|---|---|---|---|---|
| 1 | 55° | 42-27° | 10³-60 | 30,0 g | 0,0 |
| 2 | 55-72° | 25-62° | 19 | 0,5 g | 3,6 |
| 3 | 69-135° | 63-65° | 19 | 41,8 g | 97,6 |
| Rückstand | | | | 11,3 g | |

Die Hauptfraktion (3) ergibt 41,8 g (71,4% der theoretischen Ausbeute) farblose Flüssigkeit, Sdp. 63-65°C/19 mbar, welche nach Gaschromatographie zu 97,6 F% aus dem erwünschten Produkt 2-Hydroxy-2-methyl-3-butenal-dimethylacetal besteht.

### Beispiel 8

### Herstellung von 2-Acetoxy-2-methyl-3-butenal-dimethylacetal (Formel VII)

In einem mit Rückflusskühler, Septum (zur Probenahme), Tropftrichter und Thermometer versehenen 2 L-Vierhalskolben wird 1,224 kg (12 Mol) Essigsäureanhydrid mittels eines Oelbads zu kräftigem Sieden erhitzt. Dann werden 441,3 g (3 Mol) 2-Hydroxy-2-methyl-3-butenal-dimethylacetal innert einer Stunde zugegeben. Dabei wird die Badtemperatur bei 200-215°C gehalten, so dass der Kolbeninhalt weiter heftig siedet und die Temperatur am Kopf des Rückflusskühlers bei etwa 130°C bleibt.

Etwa 6 Stunden nach beendeter Zugabe des Dimethylacetals, währenddessen etwa 450 g Essigsäure-Essigsäureanhydrid entnommen worden ist, steigt die Temperatur am Rückflusskühlerkopf. Man entnimmt weitere 210 g Destillat (hauptsächlich Essigsäureanhydrid).

Der Kolbeninhalt (etwa 1 kg) wird nun flachdestilliert, und zwar bei einer Badtemperatur von 70-100°C, was etwa 997 g Destillat, Sdp. 48-85°C/20 mbar, ergibt (7-8 g Rückstand). Aus dem Destillat wird dann der restliche Essigsäureanhydrid-Ueberschuss bei 17-23 mbar über eine Kolonne mit Raschigringfüllung bei einer Badtemperatur von 90-115°C abdestilliert, was 440-442 g, aus praktisch reinem Essigsäureanhydrid bestehendes Destillat ergibt. Der schwach gelblich gefärbte Rückstand (etwa 550-555 g) enthält nur etwa 0,5% Essigsäureanhydrid und kann direkt für die anschliessende Umlagerung eingesetzt werden. Er besteht hauptsächlich (etwa zu 97%) aus dem gewünschten 2-Acetoxy-2-methyl-3-butenal-dimethylacetal, Sdp. 85-86°C/20 mbar. Die Ausbeute beträgt etwa 95% der Theorie.

### Beispiel 9

### Herstellung von γ-Acetoxytiglinaldehyd-dimethylacetal (Formel VIII)

In einem 50 ml Rundkolben, welcher mit einem Rückflusskühler und einem Calciumchloridrohr ausgerüstet ist, wird ein Gemisch von 30 g 2-Acetoxy-2-methyl-3-butenal-dimethylacetal und 0,22 g Bis-(acetonitril)palladiumchlorid unter Rühren mit einem Magnetrührer 1 Stunde bei 50°C erhitzt. Der Palladiumkomplex geht dabei in Lösung, welche anfangs gelb, am Schluss der Reaktion braunrot gefärbt ist. Der Kolben wird auf Raumtemperatur abgekühlt und der Katalysator mit einem Dünnschichtverdampfer abgetrennt (Manteltemperatur 50°C, Druck 0,01 Torr). Das erhaltene Produkt (29,8 g) besteht zu 89,6% aus trans-γ-Acetoxytiglinaldehyd-dimethylacetal und zu 8,7% aus der entsprechenden cis-Verbindung.

### Beispiel 10

### Herstellung von γ-Acetoxytiglinaldehyd (Formel I)

In einem 250 ml Vierhalskolben, ausgerüstet mit Magnetrührer, Thermometer und Tropftrichter, werden 200 ml 1N Salzsäure vorgelegt. Innerhalb von 5 Minuten werden unter intensivem Rühren 79,6 g γ-Acetoxytiglinaldehyd-dimethylacetal (0,424 Mol) zugetropft. Der Tropftrichter wird mit 10 ml Methylenchlorid gespült. Die Lösung wird 15 Minuten bei Raumtemperatur weitergerührt. Nach Abtrennung der wässrigen Phase wird diese mit 150 ml Methylenchlorid und zweimal mit jeweils weiteren 70 ml Methylenchlorid extrahiert. Die vereinigten organischen Phasen werden mit 70 ml gesättigter Natriumbicarbonatlösung gewaschen und mit 20 g wasserfreiem Natriumsulfat getrocknet. Das Lösungsmittel wird am Rotationsverdampfer abgedampft. Man erhält 61,4 g rohen γ-Acetoxytiglinaldehyd.

## Patentansprüche

1. Verfahren zur Herstellung von γ-Acetoxytiglinaldehyd, dadurch gekennzeichnet, dass man ein Pentenin-3-ol der allgemeinen Formel
HC≡C-C(CH₃)(OH)-CH=C(R)₂ II
worin beide R entweder Wasserstoff oder Methyl bedeuten, einer selektiven Ozonolyse in einem niederen Alkanol R¹OH, worin R¹ C₁₋₄-Alkyl bedeutet, unterwirft, das so erhaltene 2-Hydroxy-2-methyl-3-butinal der Formel
HC≡C-C(CH₃)(OH)-CHO III
sowie allfällig als Nebenprodukt erhaltenes 1-Alkoxyalkyl-hydroperoxid der allgemeinen Formel 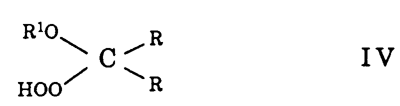 worin beide R entweder Wasserstoff oder Methyl und R¹ C₁₋₄-Alkyl bedeuten,
zum bekannten 2-Hydroxy-2-methyl-3-butenal der Formel
H₂C=CH-C(CH₃)(OH)-CHO V
katalytisch (und im Falle des 2-Hydroxy-2-methyl-3-butinals der Formel III auch selektiv) hydriert und dieses auf an sich bekannte Weise in den gewünschten γ-Acetoxytiglinaldehyd der Formel
CH₃COO-CH₂CH=C(CH₃)-CHO I
überführt.

2. Verfahren nach Anspruch 1, dadurch gekennzeichnet, dass R¹ Methyl bedeutet.

3. Verfahren nach Anspruch 1 oder 2, dadurch gekennzeichnet, dass die Ozonolyse bei Temperaturen im Bereich von etwa -20°C bis etwa 0°C erfolgt.

4. Verfahren nach einem der Ansprüche 1 bis 3, dadurch gekennzeichnet, dass man als Katalysator für die Hydrierung einen Palladiumkatalysator verwendet.

5. Verfahren nach Anspruch 4, dadurch gekennzeichnet, dass der Palladiumkatalysator Palladium auf Aluminiumoxid oder Palladium auf Kohle ist.

6. Verfahren nach Anspruch 4 oder 5, dadurch gekennzeichnet, dass die Hydrierung bei Temperaturen zwischen etwa 20°C und etwa 40°C, vorzugsweise bei Raumtemperatur, erfolgt.

7. Verfahren nach einem der Ansprüche 1 bis 6, dadurch gekennzeichnet, dass man das Butenal der Formel V sowie allfällig vorhandenes Halbacetal davon, der Formel H₂C=CH-C(CH₃)(OH)-CH(OH)(OR¹), zum entsprechenden Dialkylacetal der Formel
H₂C=CH-C(CH₃)(OH)-CH(OR²)₂ VI
worin R² C₁₋₄-Alkyl bedeutet,
acetalisiert [bzw. zum Dialkylacetal der Formel H₂C=CH-C(CH₃)(OH)-CH(OR¹)(OR²) VI' vollacetalisiert], das Dialkylacetal acetyliert, das resultierende 2-Acetoxy-2-methyl-3-butenal-dialkylacetal der Formel
H₂C=CH-C(CH₃)(OCOCH₃)-CH(OR²)₂ VII
[bzw. H₂C=CH-C(CH₃)(OCOCH₃)-CH(OR¹)(OR²)] VII'
einer katalysierten Umlagerung zum γ-Acetoxytiglinaldehyd-dialkylacetal der Formel
CH₃COO-CH₂CH=C(CH₃)-CH(OR²)₂ VIII
[bzw. CH₃COO-CH₂CH=C(CH₃)-CH(OR¹)(OR²)] VIII'
unterwirft und dieses mittels säurekatalysierter Hydrolyse in den gewünschten γ-Acetoxytiglinaldehyd der Formel I überführt.

## Claims

1. A process for the manufacture of γ-acetoxytiglic aldehyde, characterized by subjecting a pentenyn-3-ol of the general formula
HC≡C-C(CH₃)(OH)-CH=C(R)₂ II
wherein both R's signify either hydrogen or methyl, to a selective ozonolysis in a lower alkanol R¹OH, wherein R¹ signifies C₁₋₄-alkyl, catalytically hydrogenating the thus-obtained 2-hydroxy-2-methyl-3-butynal of the formula
HC≡C-C(CH₃)(OH)-CHO III
as well as any 1-alkoxyalkyl-hydroperoxide of the general formula 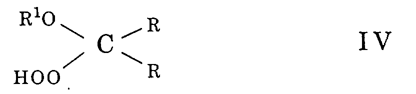 wherein both R's signify either hydrogen or methyl and R¹ signifies C₁₋₄-alkyl,
which may be obtained as a byproduct (the hydrogenation being selective in the case of the 2-hydroxy-2-methyl-3-butynal of formula III) to the known 2-hydroxy-2-methyl-3-butenal of the formula
H₂C=CH-C(CH₃)(OH)-CHO V
and converting this in a manner know per se into the desired γ-acetoxytiglic aldehyde of the formula
CH₃COO-CH₂CH=C(CH₃)-CHO I.

2. A process according to claim 1, characterized in that R¹ signifies methyl.

3. A process according to claim 1 or 2, characterized in that the ozonolysis is effected at temperatures in the range of about -20°C to about 0°C.

4. A process according to any one of claims 1 to 3, characterized in that a palladium catalyst is used as the catalyst for the hydrogenation.

5. A process according to claim 4, characterized in that the palladium catalyst is palladium on aluminum oxide or palladium on charcoal.

6. A process according to claim 4 or 5, characterized in that the hydrogenation is effected at temperatures between about 20°C and about 40°C, preferably at room temperature.

7. A process according to any one of claims 1 to 6, characterized in that the butenal of formula V as well as any hemiacetal thereof of the formula H₂C=CH-C(CH₃)(OH)-CH(OH)(OR¹) which may be present is acetalized to the corresponding dialkyl acetal of the formula
H₂C=CH-C(CH₃)(OH)-CH(OR²)₂ VI
wherein R² signifies C₁₋₄-alkyl,
[or fully acetalized to the dialkyl acetal of the formula H₂C=CH-C(CH₃)(OH)-CH(OR¹)(OR²) VI'], the dialkyl acetal is acetylated, the resulting 2-acetoxy-2-methyl-3-butenal dialkyl acetal of the formula
H₂C=CH-C(CH₃)(OCOCH₃)-CH(OR²)₂ VII
[or H₂C=CH-C(CH₃)(OCOCH₃)-CH(OR¹)(OR²)] VII'
is subjected to a catalyzed rearrangement to the γ-acetoxytiglic aldehyde dialkyl acetal of the formula
CH₃COO-CH₂CH=C(CH₃)-CH(OR²)₂ VIII
[or CH₃COO-CH₂CH=C(CH₃)-CH(OR¹)(OR²)] VIII'
and this is converted into the desired γ-acetoxytiglic aldehyde of formula I by acid-catalyzed hydrolysis.

## Revendications

1. Procédé de préparation de l'aldéhyde gamma-acétoxytiglique caractérisé en ce que l'on soumet un pentényn-3-ol de formule générale
HC≡C-C(CH₃)(OH)-CH=C(R)₂ II
dans laquelle chacun des symboles R représentent l'hydrogène ou un groupe méthyle, à une ozonolyse sélective dans un alcanol inférieur R¹OH, R¹ représentant un groupe alkyle en C1-C4, ce qui donne le 2-hydroxy-2-méthyl-3-butynal de formule
HC≡C-C(CH₃)(OH)-CHO III
et le cas échéant, en produit d'accompagnement, l'hydroperoxyde de 1-alcoxyalkyle de formule générale 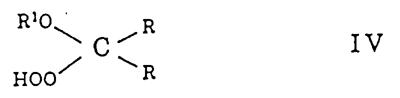 dans laquelle chacun des symboles R représentent l'hydrogène ou un groupe méthyle et
R¹ représente un groupe alkyle en C1-C4,
qu'on convertit par hydrogénation catalytique (et également sélective dans le cas du 2-hydroxy-2-méthyl-3-butynal de formule III) en le 2-hydroxy-2-méthyl-3-buténal connu de formule
H₂C=CH-C(CH₃)(OH)-CHO V
qu'on convertit, de manière connue en soi, en l'aldéhyde gamma-acétoxytiglique recherché de formule
CH₃COO-CH₂CH=C(CH₃)-CHO I

2. Procédé selon revendication 1, caractérisé en ce que R¹ représente un groupe méthyle.

3. Procédé selon revendication 1 ou 2, caractérisé en ce que l'ozonolyse est réalisée à des températures dans l'intervalle d'environ -20 à 0° C.

4. Procédé selon une des revendications 1 à 3, caractérisé en ce que le catalyseur utilisé pour l'hydrogénation est un catalyseur au palladium.

5. Procédé selon revendication 4, caractérisé en ce que le catalyseur au palladium consiste en palladium sur alumine ou palladium sur charbon.

6. Procédé selon revendication 4 ou 5, caractérisé en ce que l'hydrogénation est réalisée à des températures allant d'environ 20 à 40° C, de préférence à température ambiante.

7. Procédé selon une des revendications 1 à 6, caractérisé en ce que le buténal de formule V et le cas échéant son hémi-acétal, de formule H₂C=CH-C(CH₃)(OH)-CH(OH)(OR¹), sont acétalisés en le dialkylacétal correspondant de formule
H₂C=CH-C(CH₃)(OH)-CH(OR²)₂ VI
dans laquelle R² représente un groupe alkyle en C1-C4, et respectivement entièrement acétalisé en le dialkylacétal de formule H₂C=CH-C(CH₃)(OH)-CH(OR¹)(OR²)VI', on acétyle le dialkylacétal, ce qui donne le dialkylacétal du 2-acétoxy-2-méthyl-3-buténal de formule
H₂C=CH-C(CH₃)(OCOCH₃)-CH(OR²)₂ VII
[ou respectivement H₂C=CH-C(CH₃)(OCOCH₃)-CH(OR¹)(OR²)] VII'
qu'on soumet à transposition catalytique en le dialkylacétal de l'aldéhyde gamma-acétoxytiglique de formule
CH₃COO-CH₂CH=C(CH₃)-CH(OR²)₂ VIII
[ou respectivement CH₃COO-CH₂CH=C(CH₃)-CH(OR¹)(OR²)] VIII'
qu'on convertit par hydrolyse catalysée par un acide en l'aldéhyde gamma-acétoxytiglique recherché de formule I.
